# EUROPEAN PATENT SPECIFICATION

(11) **EP 1 885 412 B1**
(45) Date of publication and mention of the grant of the patent: **23.09.2020**
(21) Application number: 06710131.1
(22) Date of filing: 21.03.2006
(51) Int. Cl.: A61M 5/20

(54) **AN INJECTION DEVICE WITH ANGLED TRIGGER**
INJEKTIONSVORRICHTUNG MIT ABGEWINKELTEM AUSLÖSER
DISPOSITIF D'INJECTION À DÉTENTE COUDÉE

(30) Priority: 06.04.2005 GB 0507015
(43) Date of publication of application: 13.02.2008
(73) Proprietor: Cilag GmbH International, 6300 Zug (CH)
(72) Inventor: HOGWOOD, Jonathan, Cambridge CB1 7TS (GB); HABESHAW, Rosemary Louise, Hertfordshire SG8 6EQ (GB)
(74) Representative: Tunstall, Christopher Stephen
(86) International application number: PCT/GB2006/001023
(87) International publication number: WO 2006/106292

(56) References cited:
- WO-A2-00/69488
- GB-A- 2 414 402
- GB-A- 2 414 403
- US-A- 3 656 472
- US-A- 4 194 505
- US-A- 5 645 536
- US-B1- 6 454 746
- US-B1- 6 575 939

## Description

### FIELD OF THE INVENTION

The present invention relates to an injection device of the type that receives a syringe, extends it, discharges its contents and then retracts it automatically.

### BACKGROUND OF THE INVENTION

Previously known injection devices are shown in WO 95/35126 and EP-A-0 516 473 and tend to employ a drive spring and a trigger that, when activated, causes the drive spring to act on the syringe when a releasable locking mechanism is also engaged. WO00/69488 describes an injector device comprising a control button arranged on the housing, which, through a transmission and a switch system, stepwise triggers the penetration arrangement and injection arrangement, the button being arranged to have at least a movement component perpendicular to the needle axis.

The trigger acts is rotatable about an axis so that when it is depressed at a first end, a second end (which normally engages with the drive spring) is also rotated, thereby releasing the drive spring, extending the syringe and discharging its contents. The trigger is generally mounted in the side of the housing of the injection device parallel to the longitudinal axis of the body of the injection device. The trigger is rotated by pressing down on the trigger at one end in a direction towards the housing.

A problem with such devices is that the action of pressing down on the trigger towards the housing can be difficult if the device is being held in one hand. This poses significant problems for sick and ill users of the injection device, for example users suffering from rheumatoid arthritis.

### SUMMARY OF THE INVENTION

The injection device of the present invention is designed to deal with this and other problems.

In view of the foregoing and in accordance with the invention, there is provided an injection device according to claim 1 and comprising inter alia :
a housing defining a first axis, and being adapted to receive a syringe having a discharge nozzle, so that the syringe is movable between a retracted position in which the discharge nozzle is contained within the housing and an extended position in which the discharge nozzle extends from the housing through an exit aperture;
a drive that is acted upon and in turn acts upon the syringe; and
a trigger rotatable from a rest position, in which it causes the drive to be retained, to an active position, in which it no longer causes the drive to be so retained, thus allowing the contents of the syringe to be discharged through the discharge nozzle,
wherein the trigger is pivotally mounted and has a surface shaped such that a user can apply a force in a direction substantially parallel to the first axis to rotate the trigger from its rest position to its active position.

By having the trigger operable so that a force can be applied in a direction which is substantially parallel to the first axis, the trigger does not need to be pushed into the side of the housing to activate it. This way, the injection device can be held and operated with one hand by sliding the hand down the housing and over the trigger as the exit aperture is pushed against a user's body.

According to the invention, the surface is a first concave surface. In one embodiment of the invention, the surface is provided with a plurality of ridges.

According to the invention, the housing is provided with a second concave surface substantially opposite to the trigger so that a user can grip the device and apply a force to the trigger and second concave surface in a direction substantially parallel to the first axis.

In one embodiment of the invention, the housing comprises an abutment located adjacent the trigger between the trigger and the exit aperture.

In one embodiment of the invention, the second concave surface is provided with a plurality of ridges.

### BRIEF DESCRIPTION OF THE DRAWINGS

The invention will now be described by way of example with reference to the accompanying drawings, in which:
Figure 1 shows a perspective view of an injection device according to the present invention;
Figure 2 shows an alternative perspective view of an injection device according to the present invention; and
Figure 3 shows a side view of the injection device of figures 1 and 2 with an upper section of the housing of the injection device removed.

### DETAILED DESCRIPTION OF THE DRAWINGS

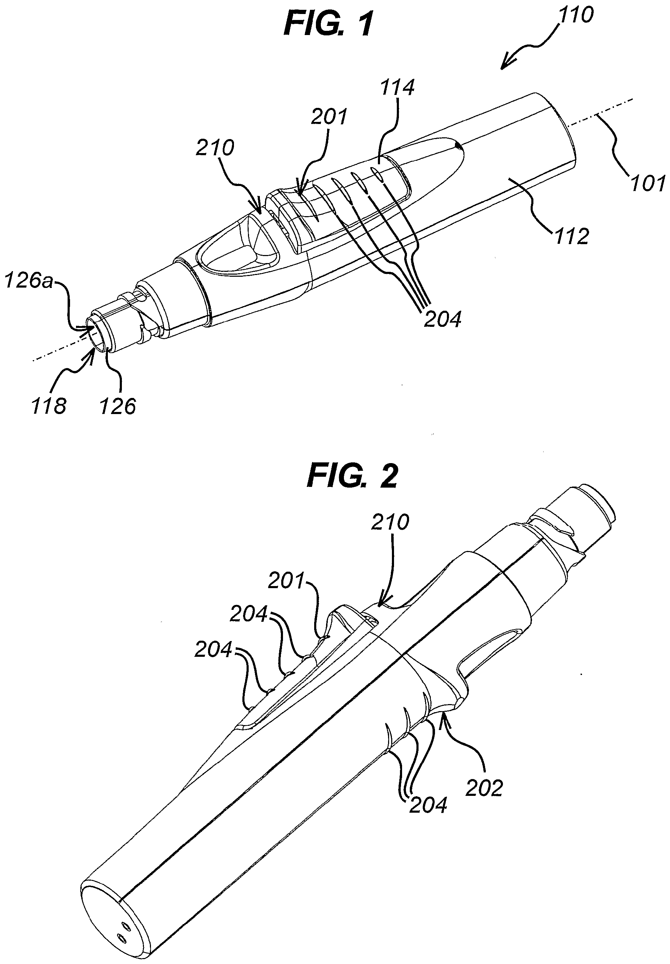

Figures 1 to 3 show an injection device 110 according to a first embodiment of the present invention. The injection device 110 has an injection device housing 112 and a longitudinal axis 101.

A syringe (not shown) is contained in the housing 112. The injection device 110 comprises trigger 114 and a releasable locking mechanism 116. The trigger 114 has a first end 114a and a second end 114b. The trigger 114 is rotatable about a pivot 115 from a rest position to an active position. The second end 114b of the trigger 114 connects with a drive coupling 121 which is acted upon by a drive spring 120. The drive coupling 121 is in communication with the syringe.

Rotation of the trigger 114 about the pivot 115 in a direction R (i.e. downwards into the housing 112 at its first end 114a) causes the second end 114b of the trigger 114 to disengage from the drive coupling 121, thereby letting the drive spring 120 drive the syringe (via the drive coupling 121) along the longitudinal axis 101 and out of an aperture 118 in the housing 112.

The releasable locking mechanism 116 is in communication with sliding sleeve 126 which protrudes, when in a first position, from the aperture 118 in the housing 112. The locking mechanism 116 is deactivated by movement of the sliding sleeve 126 along the longitudinal axis 101 into the housing 112 into a second position.

A first end 126a of the sliding sleeve 126 can be placed against a body into which drug is being delivered, thereby deactivating the releasable locking mechanism 116 and allowing the trigger 114 to rotate in direction R from its rest position to its active position.

The trigger 114 is shaped in such a way that there is a section of surface 201 at an angle to the longitudinal axis 101 of the injection device 110. Hence, rotation of the trigger 114 can take place by exertion of force in a direction which is not necessarily perpendicular to the longitudinal axis. This way, a rotational force can still be exerted on the trigger 114, even if that force is not applied inwards towards the housing 112.

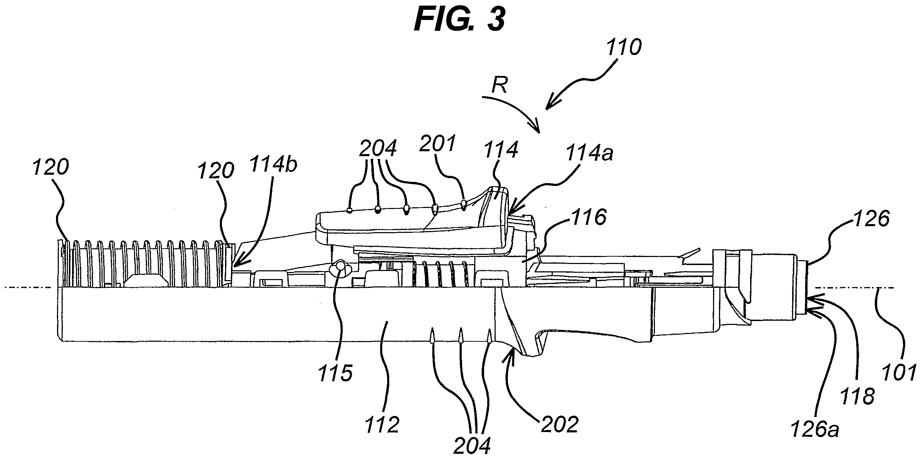

In the embodiment of the invention shown in Figures 1 and 3, the surface 201 is shaped concavely.

The hand of a user of the injection device 110 can be wrapped around the housing 112 and the injection device 110 forced against the user's body so that the sliding sleeve 126 causes the locking mechanism 116 to disengage. The trigger 114 can now be activated by movement of the user's hand across the housing 112 and over the trigger 114 so that a force having a component in the direction of the longitudinal axis 101 is applied to the trigger 114 causing it to rotate.

The housing 112 also comprises a ridge 210 (or an abutment) located behind the trigger 114 towards the exit aperture 118 of the housing 112. The ridge acts as a stop surface to prevent a user's hand moving further down the housing 112 once the trigger 114 has been activated. This way, the injection device 110 can be held firmly against a user's body after activation of the trigger 114 and whilst the contents of the syringe is being dispelled into their body.

The housing 112 comprises a second concave surface 202 located substantially opposite to the trigger 114. Thus, the user's hand is prevented from moving further down the housing 112 once the trigger 114 has been activated.

The surface 201 and a section of the surface of the housing opposite the trigger 114 both comprise ridges 204 which act as grips for the user's hand.

It will of course be understood that the present invention has been described above purely by way of example and modifications of detail can be made within the scope of the claims which define the invention.

## Claims

1. An injection device (110) comprising:
a housing (112) defining a first axis (101), and being adapted to receive a syringe having a discharge nozzle, so that the syringe is movable between a retracted position in which the discharge nozzle is contained within the housing and an extended position in which the discharge nozzle extends from the housing through an exit aperture (118);
a drive (121) that is acted upon and in turn acts upon the syringe; and
a trigger (114) rotatable from a rest position, in which it causes the drive (121) to be retained, to an active position, in which it no longer causes the drive (121) to be so retained, thus allowing the contents of the syringe to be discharged through the discharge nozzle, wherein
the trigger (114) is pivotally mounted and **characterised in that** the trigger (114) is provided with a first concave surface (201) such that a user can apply a force in a direction substantially parallel to the first axis (101) to rotate the trigger (114) from its rest position to its active position,
wherein the trigger (114) and housing (112) are arranged such that the force to rotate the trigger (114) from its rest position to its active position is applied by movement of a user's hand across the housing (112) and over the trigger (114), wherein the housing (112) is provided with a second concave surface (202) substantially opposite to the trigger (114) so that a user can apply said force to the trigger (114) and to the second concave surface (202).

2. The injection device of claim 1, wherein the first concave surface (201) is provided with a plurality of ridges (204).

3. The injection device of claim 1, wherein the second concave surface (202) is provided with a plurality of ridges (204).

4. The injection device of any one of the preceding claims, wherein a section of the surface of the housing (112) substantially opposite to the trigger (114) includes a plurality of ridges (204).

5. The injection device of any one of the preceding claims wherein the housing (112) comprises an abutment (210) located adjacent the trigger between the trigger (114) and the exit aperture (118).

## Patentansprüche

1. Injektionsvorrichtung (110), umfassend:
ein Gehäuse (112), das eine erste Achse (101) definiert und zur Aufnahme einer Spritze mit einer Austragdüse ausgeführt ist, so dass die Spritze zwischen einer zurückgezogenen Position, in der die Austragdüse in dem Gehäuse enthalten ist, und einer ausgefahrenen Position, in der sich die Austragdüse von dem Gehäuse durch eine Austrittsöffnung (118) erstreckt, beweglich ist,
einen Antrieb (121), der beaufschlagt wird und seinerseits die Spritze beaufschlagt, und
einen Auslöser (114), der aus einer Ruheposition, in der er veranlasst, dass der Antrieb (121) gehalten wird, in eine aktive Position, in der er nicht mehr veranlasst, dass der Antrieb (121) gehalten wird, drehbar ist, wodurch gestattet ist, dass der Inhalt der Spritze durch die Austragdüse ausgetragen wird, wobei der Auslöser (114) schwenkbar montiert ist, **dadurch gekennzeichnet, dass** der Auslöser (114) mit einer ersten konkaven Fläche (201) versehen ist, so dass ein Benutzer eine Kraft in eine im Wesentlichen parallel zu der ersten Achse (101) verlaufenden Richtung ausüben kann, um den Auslöser (114) aus seiner Ruheposition in seine aktive Position zu drehen, wobei der Auslöser (114) und das Gehäuse (112) so angeordnet sind, dass die Kraft zum Drehen des Auslösers (114) aus seiner Ruheposition in seine aktive Position durch das Bewegen der Hand eines Benutzers über das Gehäuse (112) hinweg und über den Auslöser (114) ausgeübt wird, wobei das Gehäuse (112) im Wesentlichen gegenüber dem Auslöser (114) mit einer zweiten konkaven Fläche (202) versehen ist, so dass ein Benutzer die Kraft auf den Auslöser (114) und auf die zweite konkave Fläche (202) ausüben kann.

2. Injektionsvorrichtung nach Anspruch 1, wobei die erste konkave Fläche (201) mit einer Vielzahl von Erhöhungen (204) versehen ist.

3. Injektionsvorrichtung nach Anspruch 1, wobei die zweite konkave Fläche (202) mit einer Vielzahl von Erhöhungen (204) versehen ist.

4. Injektionsvorrichtung nach einem der vorhergehenden Ansprüche, wobei ein im Wesentlichen dem Auslöser (114) gegenüberliegender Abschnitt der Oberfläche des Gehäuses (112) eine Vielzahl von Erhöhungen (204) aufweist.

5. Injektionsvorrichtung nach einem der vorhergehenden Ansprüche, wobei das Gehäuse (112) einen dem Auslöser benachbarten Anschlag (210) zwischen dem Auslöser (114) und der Austrittöffnung (118) umfasst.

## Revendications

1. Dispositif d'injection (110) comprenant :
un boîtier (112) définissant un premier axe (101) et apte à recevoir une seringue comportant un gicleur d'écoulement, de façon à ce que la seringue puisse se déplacer entre une position rentrée, dans laquelle le gicleur d'écoulement est contenu à l'intérieur du boîtier, et une position sortie, dans laquelle le gicleur d'écoulement sort du boîtier par une ouverture de sortie (118) ;
un entraînement (121) sur lequel on agit et qui à son tour agit sur la seringue ; et
un déclencheur (114) pouvant tourner d'une position de repos, dans laquelle il retient l'entraînement (121), à une position active, dans laquelle il ne retient plus l'entraînement (121), permettant ainsi au contenu de la seringue d'être déchargé par le gicleur d'écoulement,
dans lequel le déclencheur (114) est monté pivotant et **caractérisé en ce que** le déclencheur (114) est pourvu d'une première surface concave (201) de telle sorte qu'un utilisateur puisse appliquer une force dans une direction sensiblement parallèle au premier axe (101) pour faire tourner le déclencheur (114) de sa position de repos à sa position active,
dans lequel les déclencheur (114) et boîtier (112) sont agencés de telle sorte que la force pour faire tourner le déclencheur (114) de sa position de repos à sa position active soit appliquée par un mouvement de la main d'un utilisateur d'un côté à l'autre du boîtier (112) et sur le déclencheur (114) ;
dans lequel le boîtier (112) est pourvu d'une seconde surface concave (202) sensiblement à l'opposé du déclencheur (114) de façon à ce qu'un utilisateur puisse appliquer ladite force au déclencheur (114) et à la seconde surface concave (202).

2. Dispositif d'injection selon la revendication 1, dans lequel la première surface concave (201) est pourvue d'une pluralité de stries (204).

3. Dispositif d'injection selon la revendication 1, dans lequel la seconde surface concave (202) est pourvue d'une pluralité de stries (204).

4. Dispositif d'injection selon l'une quelconque des revendications précédentes, dans lequel une section de la surface du boîtier (112) sensiblement à l'opposé du déclencheur (114) comprend une pluralité de stries (204).

5. Dispositif d'injection selon l'une quelconque des revendications précédentes, dans lequel le boîtier (112) comprend une butée (210) située tout à côté du déclencheur entre le déclencheur (114) et l'ouverture de sortie (118).
